(19) Europäisches Patentamt
European Patent Office
Office européen des brevets

(11) Publication number: **0 120 966**
**A1**

(12) # EUROPEAN PATENT APPLICATION

published in accordance with Art. 158(3) EPC

(21) Application number: 82902750.7

(22) Date of filing: 21.09.82

Data of the international application taken as a basis:

(86) International application number: PCT/JP82/00378

(87) International publication number: WO84/01148 (29.03.84 84/09)

(51) Int. Cl.³: C 07 C 49/76
C 07 C 49/782, C 07 C 49/784
C 07 C 49/786, C 07 C 49/80
C 07 C 49/813, C 07 C 49/84
C 07 C 45/51

(43) Date of publication of application: 10.10.84 Bulletin 84/41

(84) Designated Contracting States: DE FR GB NL

(71) Applicant: MITSUI TOATSU CHEMICALS, INCORPORATED
2-5 Kasumigaseki 3-chome
Chiyoda-Ku Tokyo 100(JP)

(72) Inventor: YAMAGUCHI, Akihiro
1-1-21, Iwase Kamakura-shi
Kanagawa-ken 247(JP)

(72) Inventor: YAMAGUCHI, Keizaburo
600-1, Kamisakunobe Takatsu-ku, Kawasaki-shi
Kanagawa-ken 213(JP)

(72) Inventor: SUGIMOTO, Kenichi
105, Minezawa-cho Hodogaya-ku, Yokohama-shi
Kanagawa-ken 240(JP)

(72) Inventor: TANABE, Yoshimitsu
2070, Iizima-cho Totsuka-ku
Yokohama-shi Kanagawa-ken 244(JP)

(74) Representative: Schübel-Hopf, Ursula et al,
Strehl, Schübel-Hopf, Schulz Patentanwälte
Widenmayerstrasse 17
D-8000 München 22(DE)

(54) **PROCESS FOR PREPARING BENZOPHENONE DERIVATIVES.**

(57) Benzophenone derivatives are obtained by treating 1,1-bis(aryl)-2,2,2-trichloroethanol with alkali. The starting 1,1-bis(aryl)-2,2,2-trichloroethanol is industrially available at low cost. The alkali is preferably a hydroxide or carbonate of an alkali metal, and is used in an amount of 0.2 to 10 mol per mol of 1,1-bis(aryl)-2,2,2-trichloroethanol.

EP 0 120 966 A1

Croydon Printing Company Ltd.

SPECIFICATION

# PROCESS FOR PREPARING BENZOPHENONE DERIVATIVES

Field of the Invention :

This invention relates to a process for the preparation of benzophenone derivatives and particularly a process, which comprises treating 1,1-bis(aryl)-2,2,2-trichloroethanol of Formula I

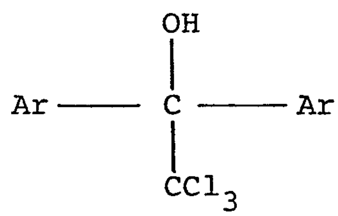

I

wherein Ar stands for a non-substituted or substituted phenyl group, with an alkali. Benzophenone derivatives may be represented by the Formula II

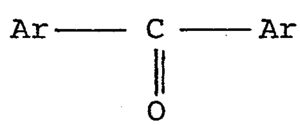

II

wherein Ar is as defined above.

Background of the Art :

Generally, benzophenone derivatives are used as agricultural chemicals, drugs and intermediates for dyestuffs and particularly, halogenated benzophenone derivatives are useful as materials for resins having good properties such as thermal resistance, oxidation resistance and light resistance.

For the preparation of benzophenone derivatives there have been known, for example, (1) a process of effecting a Friedel Crafts' reaction between an aromatic acid chloride

and an aromatic hydrocarbon in the presence of a Lewis acid catalyst such an anhydrous aluminum chloride and anhydrous ferric chloride (P.H. Gore, "Friedel Crafts and Related Reactions" Vol. III, pl, ed. by G.A. Olah, John Wiley & Sons, Inc.(1964)),(2) a process including reacting an aromatic hydrocarbon with carbon tetrachloride in the presence of anhydrous aluminum chloride and then effecting hydrolysis (J.P. Picard et al, Can. J. Research, 28B, 56 (1950) ) and (3) a process of oxidation of a 1,1-bis(4-halogenated phenyl)-2,2-dichloroethylene compound with chromic acid (H.L. Bradlow et al, J.Am. Chem. Soc., 69, 662 (1947); O. Grumitt et al, J. Am. Chem. Soc., 67, 155 (1945); O.G. Bacheberg et al, J. Chem. Soc., 1945, 803).

However, in case of process (1) anhydrous aluminum chloride in amounts equimolar to the aromatic acid chloride is required and in case of process (2) anhydrous aluminum chloride in an equimolar amount to the aromatic hydrocarbon is used. The separation of aluminum chloride is too complicated to reuse it and further, anticorrosive equipment is required. In case of process (3) it is relatively easy to convert 1,1-bis(aryl)-2,2,2-trichloroethanes to the ethylene derivatives thereof by

alkali treatment, in which the former is prepared by reaction of an aromatic hydrocarbon with chloral (for example, O.Grummitt et al, J.Am Chem. Soc., 67,155 (1945) ). It is, however, extremely troublesome to use a large amount of chromic acid in the oxidation reaction and the treatment of waste fluid generated requires much expenses. Therefore conducting the process (3) in a commercial scale is confronted with various difficulties.

Disclosure of the Invention:

An object of this invention is to provide a process for preparing benzophenone derivatives advantageously in a commercial scale.

In accordance with this invention, there is provided a process for the preparation of benzophenone derivatives which comprises treating 1,1-bis(aryl)-2,2,2-trichloroethanol with an alkali.

According to this invention, 1,1-bis(aryl)-2,2,2-trichloroethanol which is available industrially at low costs is used for the alkali treatment and as a result benzophenone derivatives can be prepared with a high yield. Therefore there are no problems of environmental pollution as compared with the conventional methods for preparing benzophenone derivatives using a large amount of aluminum chloride and chromic acid. Thus the process of this invention is very advantageous industrially.

Best Mode for Carrying Out the Invention:

The starting material used in this invention is 1,1-bis(aryl)-2,2,2-trichloroethanol which may be represented by Formula III

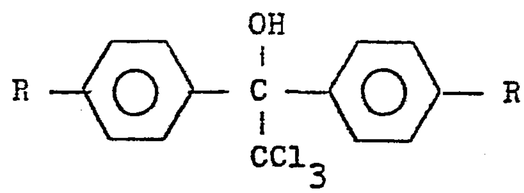

III

wherein R stands for hydrogen, halogen, an alkyl group, a phenyl group, an alkoxy group or a phenoxy group. Examples of the aryl group in Formula III include phenyl, 4-fluorophenyl, 4-chlorophenyl, 4-bromophenyl, 4-iodophenyl, 4-methylphenyl, 4-ethylphenyl, 4-isopropylphenyl, 4-t-butylphenyl, 4-phenylphenyl, 4-methoxyphenyl, 4-ethoxyphenyl, 4-butoxyphenyl and 4-phenoxyphenyl.

These 1,1-bis(aryl)-2,2,2-trichloroethanols are easily prepared by, for example, chlorinating 1,1-bis(aryl)-2,2,2-trichloroethane which is obtained by reaction between an aromatic hydrocarbon and chloral (O.Grumitt et al, J.Am. Chem. Soc., 67,155 (1945); W.Awe, Arech. Pharm., 285,162 (1950); Chem. Abstr. 45, 583k (1953) ) and hydrolysing the resulting product (US Patent No. 2,812,280 and No. 2,812,360).

The alkali which may be used in this invention includes hydroxides of an alkali metal (including ammonia) or alkaline earth metal carbonates thereof, for example sodium hydroxide, potassium hydroxide lithium hydroxide, barium hydroxide, sodium carbonate, potass carbonate and ammonium carbonate.

The alkali is generally used in the form of an aqueous solution or may be used in the form dissolved in an alcohol such as metanol and ethanol.
The amount of the alkali is within a molar ratio of 0.2 - 10, preferable 0.4 - 5 relative to 1,1-bis(aryl)-2,2,2-trichloroethanol.

In the process of this invention, the reaction may be carried out in an aqueous solution or in an organic solvent. Examples of the organic solvent include hydrocarbons such as hexane, cyclohexane, benzene, toluene and xylene, halogenated hydrocarbons such as dichloromethane, chloroform, carbon tetrachloride, dichloroethane, and chlorobenzene, alcohols such as methanol, ethanol, isopropanol and butanol, ethers such as diethyl ether, tetrahydrofuran and dioxane, ketones such as acetone and methyl ethyl ketone and ethyl acetate, N,N-dimethyl formamide and dimethyl sulfoxide and other organic solvents which are stable against an alkali.
These solvents may be used in mixture of two or more in mixture with water.

The reaction is carried out by suspending or dissolving 1,1-bis(aryl)-2,2,2-trichloroethanol in water or an organic solvent and dropping an alkali solution while maintaining at desired temperature or by dropping the solution of 1,1-bis(aryl)-2,2,2-trichloroethanol to the alkali solution or by charging the two reactants simultaneously.
The reaction temperature is comprised between 0° and 200°, preferable 10° and 150°C.

After effecting the reaction under the indicated temperature and time conditions, the resulting precipitates are filtered to obtain end products. Also, the product, when dissolved in the solvent, is charged into water and then subject to after treatment such as filtration. Simultaneously with the progress of reaction chloroform is formed, which is removed during or after the reaction.

The benzophenone derivatives obtained according to this invention are, for example, benzophenone, 4,4'-difluorobenzophenone, 4,4'-dichlorobenzophenone, 4,4'-dibromobenzophenone, 4,4'-diiodobenzophenone, 4,4'-dimethylbenzophenone, 4,4'-diethylbenzophenone, 4,4'-diisopropylbenzophenone, 4,4'-di-t-butylbenzophenone, 4,4'-diphenylbenzophenone, 4,4'-dimethoxybenzophenone, 4,4'-diethoxybenzophenoen and 4,4'-diphenoxybenzophenone.

This invention will be illustrated by the following non-limitative examples.

Example 1

16.8g(0.05 mols) of 1,1-bis(4-fluorophenyl)-2,2,2-trichloroethanol are dissolved in 20 ml of methanol. 5g(0.025 mols) of an aqueous solution of 20% sodium hydroxide are dropped at room temperature. The precipitates immediately deposit and stirring is effected at the same temperature for one hour. After filtration washing and drying, 4,4'-difluorobenzophenone is obtained with 10.5g(yield 96.5%).

Melting point 105° - 106°C

0120966

Pure product of white platy crystals is obtained by recrystallisation from benzene.

Melting point 106° - 107°C

Examples 2 - 5

1,1-bis(phenyl)-2,2,2-trichloroethanol, 1,1-bis(4-methylphenyl)-2,2,2-trichloroethanol, 1,1-bis(4-methoxyphenyl)-2,2,2-trichloroethanol, 1,1-bis(4-bromophenyl)-2,2,2-trichloroethanol, or 1,1-bis(4-chlorophenyl)-2,2,2-trichloroethanol are used instead of 1,1-bis(4-fluorophenyl)-2,2,2-trichloroethanol in Example 1. Alkalis as indicated in Table 1 are used and the reaction is carried out in the same manner as in Example 1 to obtain benzophenone derivatives. The results are set forth in Table 1.

Table 1

| Ex. No. | Alkali | Benzophenone Derivatives | Yield % | Melting Point, °C |
|---|---|---|---|---|
| 2 | Lithium Hydroxide | Benzophenone | 92 | 43 - 44 |
| 3 | Potassium Hydroxide | 4,4'-Dimethylbenzophenone | 93.5 | 92 - 93 |
| 4 | Sodium Hydroxide | 4,4'-Dimethoxybenzophenoe | 94 | 142-143 |
| 5 | Potassium Hydroxide | 4,4'-Dibromobenzophenone | 93 | 173-174 |
| 6 | Sodium Carbonate | 4,4'-Dichlorobenzophenone | 95 | 144-145 |

Example 7

38.9(0.1 mol) of 1,1-bis(4-chlorophenyl)-1,2,2,2-tetrachloroethane, 4 ml of water, 4g of a 96% sulfuric acid and 78 ml of glacial acetic acid are added together and heated under reflux (115° - 121°C) for 30 hours. After distilling off glacial acetic acid under reduced pressure, 50 ml of water are added and 30g(0.3 mols) of an aqueous 40% sodium hydroxide solution are added over about 10 minutes while maintaining the temperature at 80° - 90°C. Stirring is effected at the same temperature for one hour. During the reaction chloroform distilling off as azeotropic mixture with water is separated by means of a separator. After cooling to room temperature, filtering, washing and drying are effected.

23.2g of 4,4'-dichlorobenzophenone are obtained.

Yield 93%

Melting Point 144° - 145°C

Pure product of white flake crystals is obtained by recystallization from methanol.

Melting Point 146° - 147°C

Industrial Applicability:

As mentioned above, the starting material, 1,1-bis(aryl)-2,2,2-trichloroethanol, is available industrially at low cost and benzophenone derivatives can be prepared with high yield by treating the starting material with an alkali. Therefore there are no problems of environmental pollution as compared with the conventional methods for preparing benzophenonederi-

vatives using a large amount of aluminum chloride and chromic acid. Thus the process of this invention is very advantageous industrially.

CLAIMS :

1. A process for the preparation of benzophenone derivatives which comprises treating 1,1-bis(aryl)-2,2,2-trichloroethanol with an alkali.

2. The process of Claim 1 wherein the 1,1-bis(aryl)-2,2,2-trichloroethanol is represented by the formula

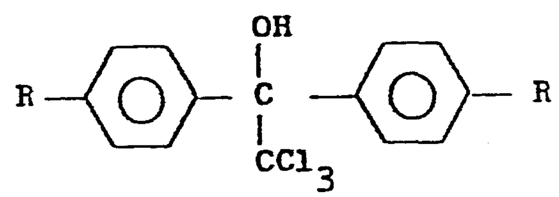

wherein R stands for hydrogen, halogen, an alkyl group, a phenyl group, an alkoxy group or a phenoxy group.

3. The process of Claim 1 or 2, wherein the alkali is a hydroxide or carbonate of an alkaline metal.

4. The process of any of the Claims 1 to 3 wherein the alkali treatment is carried out in a solution or suspension in water or in an organic solvent.

5. The process of any of the Claims 1 to 4 wherein the alkali is used in a molar ratio of 0.2 - 10 relative to the 1,1-bis(aryl)-2,2,2-trichloroethanol.

6. The process of any of the Claims 1 to 5 wherein the reaction is carried out at temperatures of 0°C - 200°C.

# INTERNATIONAL SEARCH REPORT

International Application No. PCT/JP82/00378

**I. CLASSIFICATION OF SUBJECT MATTER** (if several classification symbols apply, indicate all) [3]

According to International Patent Classification (IPC) or to both National Classification and IPC

Int. Cl.[3] C07C 49/76, 49/782, 49/784, 49/786, 49/80, 49/813, 49/84, 45/51

**II. FIELDS SEARCHED**

| Minimum Documentation Searched [4] | |
|---|---|
| Classification System | Classification Symbols |
| I P C<br>Int. Cl.[3] | C07C 49/76 - 49/84, 45/51, 45/00 |

| | Documentation Searched other than Minimum Documentation to the Extent that such Documents are Included in the Fields Searched [5] |
|---|---|
| | |

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| X | The Journal of Organic Chemistry, Vol. 46, No. 19 (1981) Faruk Nome, et al "Kinetic and Thermody — namic Study of the Base — Catalyzed Oxidative Cleavage of 1,1-Bis(p-chlorophenyl)-2,2,2-trichloroethanol" p. 3802-3804 | 1 - 6 |
| Y | Chemical Abstracts, Vol. 53, No. 17, 10. September. 1959 (10. 09. 1959) (Columbus, Ohio, U.S.A.) Ernst D. Bergmann, et al, "Bis(p-chlorophenyl)(trichloromethyl)carbinol and related compounds" 16048h-16049g, The Journal of Organic Chemistry 23 1306-8(1958) | 1 - 6 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| November 30, 1982 (30.11.82) | December 13, 1982 (13.12.82) |
| International Searching Authority [1] | Signature of Authorized Officer [20] |
| Japanese Patent Office | |